# EUROPEAN PATENT SPECIFICATION

(11) **EP 2 958 884 B1**
(45) Date of publication and mention of the grant of the patent: **24.07.2019**
(21) Application number: 14708644.1
(22) Date of filing: 18.02.2014
(51) Int. Cl.: C07C 53/02, C07C 51/15, C01B 3/06, B01J 3/00

(54) **METHOD FOR UTILIZATION OF CARBON DIOXIDE FROM INDUSTRIAL EMISSIONS INTO ENERGETIC PRODUCTS**
VERFAHREN ZUR VERWENDUNG VON KOHLENDIOXID AUS INDUSTRIEEMISSIONEN IN ENERGETISCHEN PRODUKTEN
PROCÉDÉ D'UTILISATION DE DIOXYDE DE CARBONE ISSU DE REJETS INDUSTRIELS POUR UNE TRANSFORMATION EN PRODUITS ÉNERGÉTIQUES

(30) Priority: 21.02.2013 UA 2013002213 U
(43) Date of publication of application: 30.12.2015
(73) Proprietor: Bobkov, Oleksandr Igorovich, 03179 Kiev (UA); Boiko, Anatolii Grigorovich, 01001 Kiev (UA); Bortyshevskyi, Valerii Anatoliyovich, 08304 Boryspil' (UA); Korzh, Raisa Vasylivna, 02121 Kiev (UA); Khoroshykh, Oleksii Tichonovich, 01042 Kiev (UA)
(72) Inventor: Bobkov, Oleksandr Igorovich, 03179 Kiev (UA); Boiko, Anatolii Grigorovich, 01001 Kiev (UA); Bortyshevskyi, Valerii Anatoliyovich, 08304 Boryspil' (UA); Korzh, Raisa Vasylivna, 02121 Kiev (UA); Khoroshykh, Oleksii Tichonovich, 01042 Kiev (UA)
(74) Representative: Jeck, Anton
(86) International application number: PCT/IB2014/000168
(87) International publication number: WO 2014/128546

(56) References cited:
- WO-A2-2012/090075
- US-A- 5 710 087
- US-A1- 2004 144 712
- A.A. VOSTRIKOV ET AL: "ZnO nanoparticles formation by reactions of bulk Zn with H2O and CO2 at sub- and supercritical conditions: I. Mechanism and kinetics of reactions", THE JOURNAL OF SUPERCRITICAL FLUIDS, vol. 48, no. 2, 1 March 2009 (2009-03-01), pages 154-160, XP055120044, ISSN: 0896-8446, DOI: 10.1016/j.supflu.2008.11.004
- WANG ZHENG ET AL: "Photocatalytic reduction of CO2 using Cu/S-TiO2 prepared by electroless plating method", ADVANCED MATERIALS RESEARCH; FUNDAMENTAL OF CHEMICAL ENGINEERING : SELECTED, PAPERS FROM THE 2011 INTERNATIONAL CONFERENCE ON CHEMICAL ENGINEERING AND ADVANCED MATERIALS (CEAM 2011), TRANS TECH PUBLICATIONS LTD, CH; CHANGSHA, CHINA, vol. 233-235, no. Pt. 1, 1 January 2011 (2011-01-01), pages 589-595, XP008169602, ISSN: 1022-6680, DOI: 10.4028/WWW.SCIENTIFIC.NET/AMR.233-235.589 [retrieved on 2011-05-01]

## Description

### 2. TECHNICAL FIELD

The present invention relates to fuel and power industry, namely to the application of the industrial wastes carbonic acid gas as the raw in the production of the products of energy purpose (PEP) for:
- Direct receiving of the synthetic motor fuels on the basis of methanol and formic acid;
- Direct incineration of the PEP in the HES (Heating Electric Stations) boilers for the production of warmth and electrical energy;
- Feeding of the PEP into the processes of the fine organic and petrochemical synthesis.
The application of the present invention will enable to reduce the volumes of the carbon dioxide emissions into the atmosphere owing to the utilization of the industrial wastes carbonic CO₂ acid gas and to the reduction of the level of mining and processing of the traditional fossil fuels.

### 3. BACKGROUND OF THE INVENTION

Patent WO 2007145586A1 ([1] with the analogue [2]) discloses a known method of receiving methanol as fuel for the fuel cells from the carbon dioxide and water in the fuel cell as reactor, which consists of the cathodic zone with the cathode, anodic zone with the anode and the separating membrane. The disadvantages of such method are: - the necessity of voltage application, which, at least, must be sufficient for the decomposition of water; - requirement of silver (with the adding of tellurium, ruthenium or platinum) as a catalyst of the cathode reactions; - the method is a three-fold staged one. In the description of the known Pat. [1] there is no any direct information about the operating performances of the reactor and there are no data concerning the output and selectivity as to the target products.

Pat. GB 2484095A [3] discloses a known method of the carbon dioxide utilization by the way of free radical conversion into methane and water, which includes interaction of CO₂ with hydrogen in ½ ratio under supercritical conditions of carbon dioxide (300 - 500 °C), when the process is initiated by the hydrogen peroxide, nitroxyl and perchloride radical-anions, by microwave energy, laser irradiation or by electrical field application. The following should be considered as the advantage of the method: the transformation of the carbon dioxide enhances the efficiency of the conversion process. A negative feature is that the hydrogen is used as the initial raw. Similar to the prior case there are no data about output and selectivity of the target products in the Patent description.

Patent US 2012315215A1 ([4] with the analogue [5]) discloses a known method of receiving of highly effective PEP like hydrogen, hydrocarbon gases C₁-C₅ and liquid hydrocarbon fraction without providing any specification as to their composition. In the description of this method only molecules weight distribution of hydrocarbons is presented. It is similar to the hydrocarbon distribution in the products of the Fisher-Tropsh's synthesis and that of the hydrocarbons in the famous oil fields of Russian Federation.

The method [4] and the method, as claimed, are similar as to the selection of raw (carbon dioxide as the source of carbon and water as the source of hydrogen). The authors [4] (with the analogue [5]) claim the symbiosis of two processes: Fisher-Tropsh's synthesis (interaction of CO i H₂ over the cobalt or iron catalysts under the temperature 190-240 °C and under the pressure till 0.5 MPa) and steam-gas conversion of methane (receiving of the mixture of H₂, CO and CO₂ over the nickel catalysts under the temperature 800-900 °C). As the process, according to [4], is performed in the plastic tube reactor (L = 1000_{MM}, ø = 19_{MM}) without any information about the process temperature range, it is possible to envisage, that the temperature did not exceed of 240-280 °C, and the pressure of 0.5 MPa correspondingly. In such conditions the running of steam-gas conversion of the mixture seems to be impossible. Really, the CO+H₂O ↔ CO₂+H₂ reaction displacement of equilibrium is meant here. Moreover, the hydrogen making takes place over the catalyst (iron-steam way of receiving hydrogen): 2 Fe + H₂O → Fe₂O₃ + H₂. Formed hydrogen and supplied carbon dioxide participate in the displacement of the received water gas towards the formation of CO. It is the compound, which is capable to participate in the carbon creation according to the Fisher-Tropsh's method. The formation of CO in the reactor and in the presence of water as a raw, obviously, can enable to start up the process of Fisher-Tropsh in the direction of the hydrocarbon synthesis from CO and H₂O at the temperature 210-230 °C [6], elaborated by Kolbel and Engelgardt in 1952. Nearly pure methane is received under the temperature over 300 °C. The iron catalysts with the adding of the aluminium oxides supported on the dolomite or kieselguhr are considered to be best catalysts of the method [6]. That is why the realization of the method as to the item [4] may become possible only in such an aspect.

The reason for the creation of the method [4] is the analysis of the process of the hydrocarbon synthesis in the earth's bowels. The important condition of the process's running is the presence of the considerable amount of the "free" electrons, which are formed under the natural conditions in the riff fractures of the earth crust under the mechanical impacts. The electrons renew the earth's bowels oxides of the metals to the lower levels of oxidation (even to the metallic state), which can be used as the catalysts of the CO₂ and H₂O conversion to hydrocarbon, hydrogen and oxygen.

The analysis of data on the method [4] (with the analogue [5]) demonstrates that it can be organized at the going out oil fields stratums with the purpose of increasing of the oil mining, and also during rational boring of the new oil wells. The supply of the CO₂ and H₂O mixture to them will make it possible to increase the productivity of the oil fields and to keep it stable in the process of operation owing to the production of the hydrocarbons from the carbon dioxide and water. The presented scheme of installation is a model that demonstrates the possibility of the method realization. As it was marked above, the method [4] does not include data about temperature and pressure of the process. There are no data about the rate of the process running, weight space velocity per unit of the weight or volume of the catalyst, except of the referring to the work [7] about the results of the creation of 200 cm³ of gas and 20 g of the liquid hydrocarbons from CO₂ and water for the tens of 24 hours of the experiment.

So, the method patented in [4] (with an analogue [5]), for its application requires a definite type of oil or gas fields, their prior preparation and elaboration and activation of the pot-hole areas. Such preparation requires substantial capital investments with the high risk level. The latter may be reduced owing to the boring of the super deep wells (deeper than 10 km). In the pot-hole areas of such wells, at the diapiric folds, over the reduced silicon, magnesium and iron complexes, the running of the processes of obtaining hydrogen from water is possible. The latter is directed to the oil accumulating cavities for the interaction with ₃ CO₂. Estimated duration of the method is more than 40 years with the low level of control and possible negative result.

The method, as claimed, is directed to the transformation of the mixture CO₂ and H₂O into hydrogen and oxygen consisting hydrocarbons (like formic acid), and it is based on the principally different approach. The method, as claimed, envisages the involving to the catalysis of the interaction of the carbon dioxide and water, not of "free" electrons, but positive ions (protons and cations), this, in the supercritical conditions on CO₂ + H₂O, will provide the formic acid formation (CH₂O₂). The creation of such conditions makes it possible to approach such a level of rate of target products formation, which corresponds to the appropriate technological time of contact with the outgoing components, in doing so, the used reaction volumes are small, capital costs are lower and a recoupment term is short.

The method on the closest analogue [8], which is the nearest one to the proposed approach of the carbon dioxide and water conversion as to the technical results, makes it possible to receive gas from the hydrocarbon and water under the temperature 390-908 °C (mostly over 440 °C) and under the pressure till 1.92 MPa. The gas consisting of C₁-C₂ hydrocarbons, hydrogen, carbon monoxide and oxygen, is obtained with the productivity to 750 sm³/min over catalytic complexes, which contains of CO₂ in the micellar capsular of the aqueous sol with the nucleus from Mg(CO₃)₄-Mg(OH)₂×5H₂. These catalytic complexes are prepared in a separate reactor according to the recipe on a specific patent [9]. Disadvantages of the closest method are: - high temperature of the process; - two-staged way;- difficulty in preparation; - impossibility of the catalytic complex regeneration.

A.A. Vostrikov et al. J Supercrit. Fluids 48(2009), pages 154-160 discloses the reaction between CO₂ and water under supercritical conditions using a ZnO catalyst.

### 4. DISCLOSURE OF THE INVENTION

The method, as claimed, is directed to the removal of the above mentioned disadvantages of the closest method [8]. The goal of the invention is one stage non-stop receiving of the energetic products like formic acid, formaldehyde, methanol and hydrogen by the interaction of carbon dioxide and water at the temperature of 280 to 295°C and under the supercritical pressure of 27 MPa over highly productive heterogeneous catalysts. *The technical result,* received during the embodiment of the invention, is the following:
1. Substantial reduction of ecologically harmful emissions of the carbon oxides into the environment owing to their utilization,
2. Obtaining of the new source of energy in a form of the formic acid, methanol and hydrogen.
3. Obtaining of the carbon raw for the production of the blanching materials, solvents (the monomers production), preserving additions (agricultural fodder) and poisonous chemicals (elimination of pests), and also obtaining of the plastic and deepening of the oil refining into the light oil products.

The basis of the proposed method of the carbon acid gas utilization and obtaining of the liquid energetic products is the low temperature process of the carbonatation of the active centers of the catalyst by the carbon dioxide, that results in the formation of the carbonate complex, which in the presence of water can be transformed into a formate complex. This complex can be transformed into the formic acid HCOOH or methanol CH₃OH. The fixed aim and technical result of the invention are reached owing to that fact, that water saturated by the carbon dioxide is supplied at the temperature 280-295°C and under the pressure 27 MPa till the solid catalytic contact in the form of the composite membrane from the mixture of the oxides of copper, zinc and aluminium activated by the oxides of titanium, lead, barium and niobium supported on a carrier stable in the gas and aqueous media. The received products (formic acid, formaldehyde, methanol, hydrogen, methane - examples 1-4) are separated in appropriate sections. The description of these sections is not included into the invention object. It is known, that the by-products of the formate complex decomposition can also be methyl formate, acetone, methane, ethane [10].

### 5. DRAWINGS

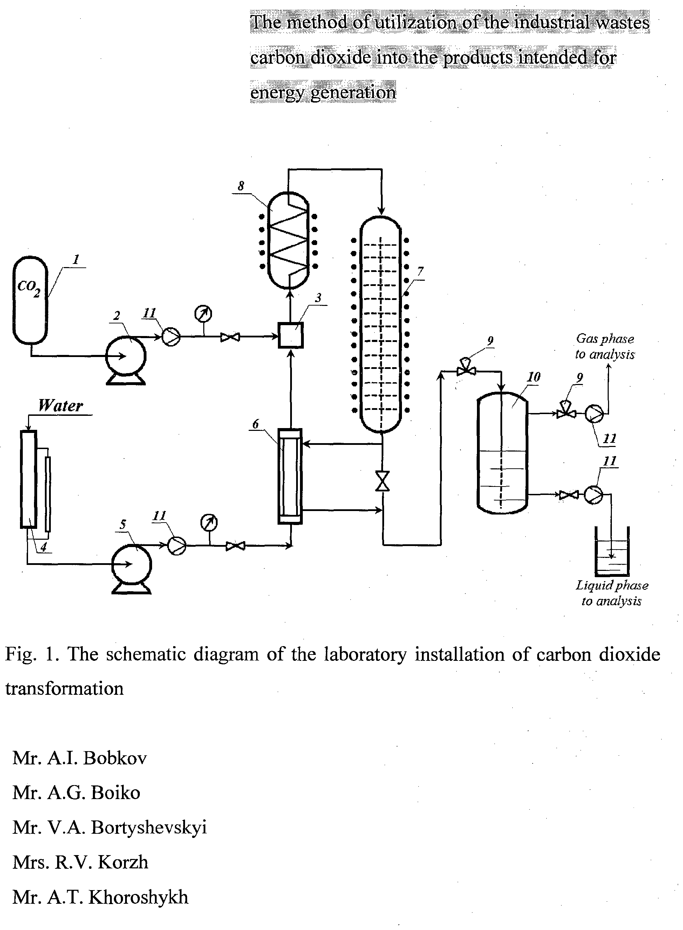

Fig. 1. The schematic diagram of the laboratory installation of carbon dioxide transformation

### 6. DETAIL DESCRIPTION OF THE INVENTION

The schematic diagram of the laboratory installation of the method, as claimed, is presented at the Fig. 1. The carbon dioxide from the gasholder (*1*) is pressed in the supercharging (forcing) compressor (2) and supplied to the mixer (3) with the specified gas flow rate, so as to be mixed with the desalted water. The water from the tank (4) is supplied with the specified flow rate by the high pressure pump (*5*) and heated in the heat exchanger (6) by the products going out from the reactor (7). The mixture received in the mixer (3) is heated in the electric oven (8) to the programed temperature (280-295°C) than it goes to the reactor (7).

The reactor (7) is a hollow cylinder made of the stainless steel 300 mm long and with the internal diameter of 28 mm, where the perforated tube is located in the center, which serves as a collector of the formed products and of unconverted raw. The layers of the composed catalyst on the thermo resistant carrier are located coaxially to the central collective tube. When the reactor is constructed this way, the going out reactive mixture moves horizontally from the reactor's walls, passes between the catalytic layers thus reacting, than it is born into the general collector, thus it goes out of the reactor. The products are cooled down by the initial water in the heat exchange (6), throttled till the atmosphere pressure by the valve (9) and come to the separator (*10*) for the separation of the gas and liquid phases. The amount of these phases is determined by the flow meters (11), while their composition is analyzed by the chromatography method.

The realization of the proposed method in comparison with the closest analogue [8] is demonstrated by the examples given in the table with description, presented below. In all investigations the distilled water with the flow rate of 33±0.2 g/hour and the carbon dioxide of high sort with the flow rate of 1.5±0.05 g/hour were used as the initial raw. The catalyst with the weight of 0.662g has been investigated without reloading.

The technological parameters and the results of experiments according to the method, as claimed, with the closest analogue are shown in the Table in comparison. The catalyst efficiency as a rate of carbon dioxide transformation in terms of elementary carbon is considered to be the main evaluating parameter. Such recalculation is introduced so as to simplify the comparison of the results according to the method, as claimed, with the closest analogue, in accordance to which the carbon is supplied to the reactor not only from the initial CO₂, but also as a component of the catalytic complexes, associated with the magnesium carbonates in a process of their preparation by way of carbonatation, added to reactor mixture as a promoter of calcium carbonate type (example 1, sample 1 according to the closest analogue [8]), introduced into the reactor mixture in the coal composition as a carrier (example 2, sample 3 according to the closest analogue), than it can pass into the target products. This made the evaluation of the catalyst efficiency according to the closest analogue much more difficult and it made the output of the carbon containing products substantially higher. Bringing (matching) of the indices to the total introduced carbon made it possible to bring to balance the known data according to the closest analogue [8] and to collate them correctly with the obtained results according to the method, as claimed. The conversion of the water transformation, which serves as a second reagent and supplied into the reactor in extra amount, has been evaluated according to the hydrogen content in the gas phase and in analyzed hydrogen containing products of the liquid phase.

### Example 1 (not according to the invention).

The carbonic acid gas from the gas holder (item *1* Fig. 1) was pressed till 24.0 Pa by the compressor (2) and supplied to the mixer (3) so as to be mixed with the distilled water, which from the tank (2) was surcharged by the compressor (5) under the same pressure. The ratio of the used carbon dioxide and water was kept 6.5 and 140 g/hour, according to the table (table, column 3, lines 6 and 7), the process was controlled by the flow meters (4). The compressed water first was preheated up in the heat exchanger (6) by the mixture of the products and unconverted raw, which went out of the reactor (*7*). The mixture CO₂+H₂O from the mixer (3) was supplied into the electrical oven (8), where it was heated up till the temperature 240 °C (table 1, column 3, line 15), which was controlled with the electric oven (*8*). The heated mixture from the oven was supplied downwards to the reactor (7, with the additional oven for maintaining temperature) to the catalyst layer located on the flexible carrier, in the direction from the reactor's wall towards the center, where the collecting tube was installed. By this tube the reaction products and unconverted raw were passed to the heat exchanger (6). After cooling down and throttling by the pressure controller (9) the products were supplied into the separator (10). The investigation time was 252 minutes. After the reaction water supply was stopped; the heating ovens were switched off; the reactive system was cooled and sealed in CO₂ atmosphere. After that the catalyst could be used in the further experiments without reloading.

During the experimental investigations gas and liquid phases were sampled from the separator (10), if necessary these phases were reduced to normal conditions, their quantitative and qualitative composition were determined at appropriate chromatographs. The obtained analytical data as to the gas and liquid composition are presented at the table (column 3, lines 28-37).

### Example 2 and 3.

In the examples 2 and 3, unlike the example 1, the temperature was increased to 270 and 280 °C, while investigation time made up 240 and 288 minutes respectively. The pressure in the reactor in both examples reached 27.0 MPa. The catalyst (without reloading) and flow rates of the CO₂ and H₂O were analogues those described in the example 1.

The obtained analytic data of the gas and liquid phases are presented in the table in the columns 4 and 5 respectively.

*Example 4.* In the example 4, unlike the examples 2 and 3, the temperature of the reaction mixture was increased to 295 °C and the reaction time was 318 minutes. As to the pressure, it remained analogues to the examples 2 end 3 (approached to 27 MPa). The catalyst (without reloading) and flow rates of the CO₂ and H₂O were analogues to those described in the example 1.

The obtained analytical data of the gas and liquid phase are presented in the table (column 6).

So, the examples 1-4 demonstrate the possibility of the realization of carbon acid gas conversion in the water medium over the catalyst on the basis of the oxides of the metals Cu, Zn, Al supported on the hard or flexible carrier in the form of the membranes, activated by the titanium, barium, lead and niobium oxides. The highest specific productivity of such catalyst for the carbon transformation at the temperature 280-295 °C under the pressure to 27 MPa, reaches about 0,06 g/(g_{cat}·h), compared with the productivity of the analogue, presented in the table (column 8 line 50) and obtained under the temperature 460 °C during 150 minutes.

Especially, it is necessary to underline, that in the presented examples the trend of the method towards the formation of the liquid products is exactly shown, especially as concerns the formic acid, formaldehyde and methanol (with the selectivity of 95-99 %) unlike the closest analogue, where CO₂ is practically entirely transformed into the gas products like CO, methane and ethane.

The comparison of the examples 2, 3 and 4 demonstrates that the higher the temperature the bigger the catalyst activity as concerns both the gas products and the target liquid products formation, the formic acid, in particular. The ratio of the carbon content in the gas and liquid phases increases to some extend under the higher temperature, it is caused by the acceleration of the methane formation process. Likely to the temperature function of the methane formation rate, the rate of the hydrogen formation increases with temperature increase. The highest productivity of hydrogen according to the method, as claimed, is reached 0.05-0.06 g/(g_{cat}·h) at the temperature 295 °C (example 4). The obtained index on it's absolute value is to some extend better than the corresponding indices for the example 2 (sample 3), according to the closest analogue (table, column 8, line 54) and it is much more worse for the example 1 (sample 1, column 7, line 54). But the substantial difference in the efficiency of the catalyst in the carbon transformation according to the closest analogue [8] is a very significant and is explained by the fact, that the authors referred CaCO₃ according to the closest analogue to the coreagent (example 1) and the coal - to the carrier (example 3). Though such doping of the active complex during the carbonation process *in situ* results in the additives like coal and CaCO₃ is transferred into the rank of the components of the active catalytic complex, as they also participate in the carbonatation. In the case, when they aren't taken into account, the efficiency calculation gives slightly higher absolute values.

When the investigations (examples 1-4) were put into practice, according to the method, as claimed, it was marked, that the catalyst activity reduction was not present during the experimental term. It enables us to envisage the term of the catalyst work without regeneration is at least a year, unlike the method according to the closest analogue [8], where the work of the catalytic complex has three stages: the increment of the efficiency in the transformation; the maximum efficiency; breaking up of the catalytic complex with the loss of the activity.

The comparative analysis of the experimental investigations results of the conversion of the carbon dioxide by water at the described installation with the data of the closest analogue US5710087 demonstrated that the method according to the closest analogue and the method, as claimed have their common characteristics:
- Both methods are intended for the carbon acid gas processing with the usage of water as the source of hydrogen;
- Both method envisage the applications of catalytic systems including those supported on the carriers;
- In both cases the authors, so as to explain the composition of the obtained products, adhere to the formate mechanism of the chemical transformations, which run on the catalytic surface. The marked common characteristics were the reasons of choosing of the Patent US5710087 as the closest analogue.

At the same time, the numbers of differentiating features were also revealed between the method according to the closest analogue and the method, as claimed:
- The main distinction is the difference of the qualitative and quantitative compositions of the obtained products. So, the gas phase from the separator, according to the method, as claimed, contains in its compositions unconverted carbon acid gas (CO₂) and hydrogen, while the gas phase, according to the closest analogue is composed primarily from the carbon monoxide (CO), methane and hydrogen with the substantial quantity of low hydrocarbons and hydrogen sulfide. The target product of the liquid phase from the separator, according to the method, as claimed, is the formic acid with the selectivity of more than 95 %. The authors of the closest analogue just indicated to the presence of the water-soluble compounds of magnesium and calcium in the liquid phase;
- The important difference in the composition of the obtained products determines another differentiating feature - that is in the further usage of the products. In particular, the gas phase, which according to the closest analogue, is the target one, and contains CO, CH₄ i H₂, is positioned as the synthetic gas raw for various chemical productions. The obtained liquid phase, which is the target one, according to the method, as claimed, after the separation stage, provides readymade marketable goods, which don't require further processing;
- The formulated difference in the composition of the gas and liquid phases, according to the closest analogue and to the method, as claimed, is first of all, explained by the differences in the nature of the used catalysts. Notwithstanding the statement of the authors of the closest analogue concerning the fact, that the CO₂ conversion by the method, according the closest analogue, is realized on the basis of formate mechanism;
- The application of the catalysts according to the method, as claimed, makes it possible to decrease the temperature regimes. So, according to the closest analogue, the range of the operating temperature makes up 440-460 °C, while in the method, as claimed, it makes up 280-295°C;
- A dramatic difference between the closest analogue and the method, as claimed, is the availability of the stage of the direct preparation of the catalytic complex in the first case and the usage of the heterogeneous catalyst during a year with the possibility of its regeneration next year;
- The usage of the heterogeneous catalyst makes it possible to switch the conversion process into the non-stop regime according to the method, as claimed, unlike half periodical regime according to the closest analogue;
- The method according to the closest analogue and the method, as claimed, considerably differ in the pressure within the reaction equipment: in the method according to the closest analogue the pressure does not exceed 1.9 MPa, and in the method, as claimed, the pressure is 27 MPa. The high pressure provides decrease of the reaction equipment size;
- The defined values of the temperature and pressure provide the running of the process under supercritical conditions for the carbon dioxide and subcritical conditions for water;
- The difference in technological parameters explains the differences in the phases system in the reactor: the method, according to the closest analogue, can be referred to the classic vapor-phase one, and as to the method, as claimed, it can be referred to the gas-liquid-solid-phase one.
- Unlike the closest analogue, the method, as claimed, is characterized by the absence of the wastes, which require additional processing or burial.
Mr. A.I. Bobkov
Mr. A.G. Boiko
Mr. V.A. Bortyshevskyi
Mrs. R.V. Korzh
Mr. A.T. Khoroshykh

### Reference

1. Pat. WO 2007145586A1, IPC C07C 29/159, C07C 29/136, C07C 31/04, C07C 47/04, C07C 29/14, C07C 45/66, C07C 45/66, C07C 53/02, C25B 9/18, B01J 21/06, B01J 21/08, B01J 21/18, B01J 23/42, B01J 23/46, B01J 23/50, B01J 27/057. A Method and a Reactor for Making Methanol: Pat. WO 2007145586, IPC C07C 29/159, C07C 29/136, C07C 31/04, C07C 47/04, C07C 29/14, C07C 45/66, C07C 45/66, C07C 53/02, C25B 9/18, B01J 21/06, B01J 21/08, B01J 21/18, B01J 23/42, B01J 23/46, B01J 23/50, B01J 27/057. Dahlberg O., Larsson A.; Morphic Technologies Aktiebolag (Pbl.). - Appl. # PCT/SE2007050418; Appl. 14/06/2007; Publ. 21/12/2007. - 17 p.
2. Pat. US 20090246572A1, Int. Cl. H01M 8/04, H01M 8/18 (2006.01). Method and a Reactor for Making Methanol: Pat. US 20090246572, Int. Cl. H01M 8/04, H01M 8/18 (2006.01). Dahlberg O., Larsson A.; Morphic Technologies Aktiebolag (Pbl.). - Appl. # 12/303349; Appl. 14/06/2007; Publ. 01/10/2009. - 4 p.
3. Pat. GB 2484095A, Int. Cl. C07C 1/12, C01B 31/20 (2006.01). CO₂ Recovery Method: Pat. GB 2484095, Int. Cl. C07C 1/12, C01B 31/20 (2006.01). Løvda K.; Løvda K.. - Appl. # 1016345.9; Appl. 29/09/2010; Publ. 04/04/2012. - 8 p.
4. Pat. US 2012315215A1, Int. Cl. C01B 3/04, C01B 13/02, C07C 1/02 (2006.01). Method of Hydrocarbons and Hydrogen Production from Water and Carbon Dioxide: Pat. US 2012315215A1, Int. Cl. C01B 3/04, C01B 13/02, C07C 1/02 (2006.01). Barenbaum A.A., Zakirov S.N., Serebryakov V.A; Galadigma LLC. - Appl. # 13/578222 Appl. 26/12/2011; Publ. 13/12/2012. - 7 p.
5. Pat. WO2012090075A2, IPC C01G 2/00 (2006.01). Method of Hydrocarbons and Hydrogen Production from Water and Carbon Dioxide Pat. WO2012090075A2, IPC C01G 2/00 (2006.01). Barenbaum A.A., Zakirov S.N., Zakirov E. S., Serebryakov V.A; Galadigma LLC. - Appl. # PCT/IB2011/003347, Appl. 26/12/2011; Publ. 05/07/2012. -23 p.
6. 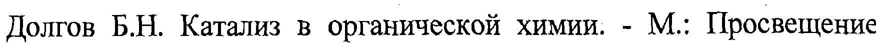 ; 1959. - 810 c.
7. 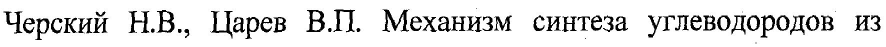 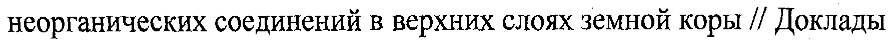 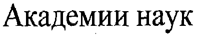 CCCP. - 1984. - T. 279, Nº 3. - C. 730-735.
8. Pat. US5710087 Int.Cl.⁶ B01J 23/02, C10G 1/00, C01B 31/18, C01B 13/02. Process for Catalytic Conversion of Water and Carbon Dioxide to Low Cost Energy, Hydrogen, Carbon Monoxide, Oxygen and Hydrocarbons: Pat. US5710087 Int.Cl.⁶ B01J 23/02, C10G 1/00, C01B 31/18, C01B 13/02. Swanson R.C.; Swanson R.C. - Appl. 476199, Appl. 07/06/1995; Publ. 20/01/1998. - 7 p.
9. Pat. US5516742A, Int.Cl.⁶ B01J 23/02. Catalytic Conversion of Water and Carbon Dioxide to Low Cost Energy, Hydrogen, Carbon Monoxide, Oxygen and Hydrocarbons: Pat. US5516742A, Iin.Cl.⁶ B01J 23/02. Swanson R.C.; Swanson R.C. - Appl. 313433, Appl. 27/09/1994; Publ. 14/05/1996. - 6 p.
10.O 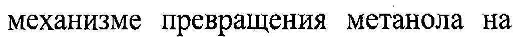 ZrO₂ 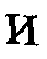 5 % Cu/ZrO₂ no 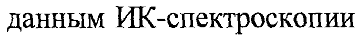 in-situ // 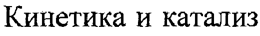 . - 2010. - T. 51, 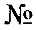 3. - C. 447-456.
   http://ru.wikipedia.org/wiki/ 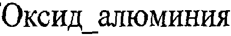 .

**Table - Comparative table of the processes' parameters on the method, as claimed, and the closest analogue [8]**

| *Indices* | | | 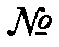 | *Claimed Method* | | | | *The closest analogue[8]* | |
|---|---|---|---|---|---|---|---|---|---|
| | | | | *Example 1* | *Example 2* | *Example 3* | *Example 4* | *Example 1 (sample 1)* | *Example 2 (sample 3)* |
| *1* | | | *2* | *3* | *4* | *5* | *6* | *7* | *8* |
| Initial reagents | | | *1* | water + carbon dioxide | | | | water + carbon dioxide | |
| Catalyst | | | *2* | CuO/ZnO | | | | Mg(CO₃)₄-Mg(OH)₂×5H₂O | |
| Activator | | | *3* | PbO, TiO₂, ZrO₂, Ni₂O₅ | | | | CaCO₃ + K₂HPO₄ | K₂HPO₄ |
| Co-reagent | | | *4* | - | | | | - | Coal Illinois #5/6 |
| Carrier | | | *5* | Al₂O₃ | | | | Al₂O₃ | - |
| Mass of components or the reaction mixture, g | Water | | *6* | 139.23 | 132.6 | 159.12 | 175.7 | 818.93 | 904 |
| | Carbon dioxide (gas) | | *7* | 6.439 | 5.746 | 6.941 | 7.75 | 4.71 | To saturation |
| | Catalyst | Produced | *8* | 0.662 | | | | 100 | 100 |
| | | Consumed | *9* | 0.662 | | | | 12.21 | 31.6 |
| | Activated | Produced | *10* | 0.066 | | | | 5+2 | 1 |
| | | Consumed | *11* | | | | | 0.86 | 0.32 |
| | Catalyst + activator | | *12* | 0.728 | | | | 13.07 | 31.92 |
| | Coal | | *13* | - | | | | - | 453.6 |
| | Carrier | | *14* | 0.126 | | | | 101.16⁽¹⁾ | - |
| Temperature, °C | | | *15* | 240 | 270 | 280 | 295 | 440 | 460 |
| Pressure, MPa | | | *16* | 23.76 | 26.86 | | | 1.93 | 1.92 |
| Process | | | *17* | Heterogeneous | | | | Vapor phase | |
| Regime | | | *18* | continuous-flow | | | | semi-flow | |
| Reaction duration | | Hour | *19* | 4.2 | 4 | 4.8 | 5.3 | 0.67 | 2.5 |
| | | Minutes | *20* | 252 | 240 | 288 | 318 | 40 | 150 |
| Coal, introduced into the reactor with the initial raw, g, | | From carbon dioxide(gas) | *21* | 1.756 | 1.567 | 1.893 | 2.114 | 1.29 | Unknown |
| | | From catalyst | *22* | - | - | - | - | 18.27 | 18.27 |
| | | From promoter | *23* | - | - | - | - | 0,6 | - |
| | | From coal | *24* | - | - | - | - | - | 199.5⁽²⁾ |
| | | *Total* | *25* | 1.756 | 1.567 | 1.893 | 2.114 | 20.16 | 217.77⁽³⁾ |
| Coal feed rate, | | g/hour | *26* | 0.418 | 0.392 | 0.394 | 0.399 | 30.09 | 87.11 |
| | | mg/mine | 27 | 6.968 | 6.529 | 6.573 | 6.648 | 504 | 1452 |
| Products | | Gas phase volume, dm³ | *28* | 2.8 | 2.15 | 2.99 | 3.38 | 12 | 80 |
| | | Mass of the liquid phase, g | *29* | 132.17 | 98.692 | 138.43 | 147.58 | Unknown | 123.1 |
| Gas phase composition, %, o6. | | Carbon dioxide | *30* | 98.3 | 87.5 | 83.2 | 83.1 | 4.76 | 84.98 |
| | | Carbon monoxide | *31* | 0.001 | 0.05 | 0.03 | 0.035 | 25.17 | 0.95 |
| | | methane | *32* | 0.002 | 0.118 | 0.228 | 0.405 | 13.29 | 3.67 |
| | | Ethane | *33* | 0.001 | - | - | - | 0.34 | 0.89 |
| | | Ethene | *34* | - | - | - | - | - | 0.18 |
| | | Hydrogen | *35* | 0.187 | 0.328 | 0.743 | 1.41 | 21.13 | 6.28 |
| | | hydrogen sulfide | *36* | - | - | - | - | - | 1,03 |
| | | air, oxygen, others ⁽⁴⁾ | *37* | 1.51 | 12.054 | 15.829 | 15.0385 | 35.31 | 2.02 |
| The liquid phase components | | | *38* | Water, methane acid, methanol, formaldehyde | | | | water, water-soluble compounds of magnesium & calcium | |
| Coal consisting of gas products, g | | In carbon dioxide | *39* | 1.475 | 1.008 | 1.332 | 1.505 | 0,306 | 133,54 |
| | | In carbon monoxide | *40* | 0.00015 | 0.00057 | 0.00048 | 0.0006 | 1.618 | 0.95 |
| | | In methane | *41* | 0.00003 | 0.00136 | 0.0037 | 0.0073 | 0.854 | 2.097 |
| | | In ethane | *42* | - | - | - | - | 0.022 | 0.954 |
| | | In ethene | *43* | - | - | - | - | - | 0.18 |
| Coal consisting of liquid products, g | | Formic acid | *44* | 0.05039 | 0.1214 | 0.1688 | 0.1872 | - | - |
| | | Methanol | *45* | traces | 0.0008 | 0.0012 | 0.0014 | - | - |
| | | Formaldehyde | *46* | 0.001 | 0.0012 | 0.0010 | 0.0008 | - | - |
| Total weight of coal in products, g⁽⁵⁾ | | | *47* | 0.0514 | 0.1253 | 0.1751 | 0.1974 | 2.494 | 4.181 |
| Average rate of coal transformation in the products | | g/h | *48* | 0.0122 | 0.0313 | 0.0365 | 0.0372 | 3.722 | 1.672 |
| | | mg/min | *49* | 0.2041 | 0.5222 | 0.6081 | 0.6207 | 62 | 27.8 |
| Catalyst efficiency in carbon transformation | | g/(g_{cat}·h) | *50* | 0.0185 | 0.0473 | 0.0551 | 0.0563 | 0.285 | 0.052 |
| | | mg/(g_{cat}·min) | *51* | 0.3083 | 0.7889 | 0.9186 | 0.9375 | 4.744 | 0.871 |
| Speed of the hydrogen creation | | g/h | *52* | 0.0005 | 0.0006 | 0.0020 | 0.0043 | 0.337 | 0.090 |
| | | mg/min | *53* | 0.0078 | - 0.0105 | 0.0331 | 0.0709 | 5.65 | 1.49 |
| Catalyst efficiency in hydrogen creation | | g/(g_{cat}·h) | *54* | 0.0007 | 0.0010 | 0.0029 | 0.0064 | 0.026 | 0.0028 |
| | | mg/(g_{cat}·min) | *55* | 0.0118 | 0.0159 | 0.0499 | 0.1071 | 0.432 | 0.047 |
| Productivity of hydrogen production, mole/mole of converted coal | | | *56* | 0.23 | 0.128 | 0.63 | 0.68 | 0.565 | 0.326 |
| Total coal conversion %, mass. | | | *57* | 2.93 | 7.99 | 9.25 | 9.34 | 12.37 | ≤1.92 |
| Selectivity on the formic acid, % | | | *58* | 99.91 | 98.45 | 97.64 | 95.96 | - | - |

| | | | | | | | | | |
|---|---|---|---|---|---|---|---|---|---|
| Notes: 1. Density of the aluminium oxide is accepted equal to 3.99 g/cm³ [11] ; 2. As estimated the carbon content in the coalI Illinois #5/6 makes up 44 %, mass; 3 .The value is lowered due to unknown weight of the carbon from the carbon dioxide; 4 The losses are included; 5 Except of CO₂ as an initial reagent. | | | | | | | | | |

## Claims

1. A method for the carbonic acid gas utilization by the way of the catalytic carbonatation of aqueous solutions comprising:
- the compressing of initial carbon dioxide to the pressure higher than the critical value,
- mixing the compressed carbon dioxide with the supercritical water,
- heating the mix to the supercritical temperature, of 280 to 295 °C,
- then the mix's feeding in a continuous mode into the reactionary facility consisting of the heterogeneous catalyst on the basis of the copper, zinc, and aluminium oxides, supported on a hard or flexible carrier in the form of a membranes and activated by lead, zirconium, titanium and niobium oxides,
- carrying out the conversion of carbon dioxide by water at a constant temperature of 280-295 °C and a constant pressure of 27 MPa, resulting in formic acid as s target product of the liquid phase from a separator with the selectivity of more than 95 %.

2. The method according to claim 1, wherein the receiving of the liquid products is followed by the generation of hydrogen and methane in amount of 0.63-0.68 and 0.07-0.123 mole per mole per mole of the converted carbon.

3. The method according to claim 1, wherein the heterogeneous catalyst on the basis of the copper, zinc and aluminum oxides in mass ratio (50-63)/(35-20)/(2-30), preferably (50-56)/(28-24)/(16-26), supported on the hard or flexible carrier in the form of the membranes and activated by the lead, zirconium, titanium, and niobium oxides in the following ratio (50-65)/(20-30)/(20-30)/(0,5-4,5), preferably (58-60)/(20-22)/(15,5-20)/(2-2,5).

4. The method according to claim 1, wherein the carbon dioxide aqueous solution with mass concentration of the carbon dioxide 3-12 % is supplied to the reactionary facility in the mass ratio of water /carbon dioxide /catalyst (20-50)/(0.5-4)/1.

## Patentansprüche

1. Verfahren zur Verwendung von Kohlensäuregas mittels der katalytischen Karbonisierung von wässrigen Lösungen, das Folgendes enthält:
- der Druck des anfänglichen Kohlendioxids ist höher als der kritische Wert,
- Mischen des unter Druck gesetzten Kohlendioxids mit superkritischem Wasser,
- Erhitzen der Mischung auf die superkritische Temperatur von 280° auf 295°C,
- Einspeisen der Mischung in kontinuierlicher Weise in eine Reaktionsvorrichtung, die aus einem heterogenen Katalysator auf der Basis von Kupfer, Zink und Aluminiumoxiden, die auf einem festen oder flexiblen Träger in der Form von Membranen getragen und durch Blei, Zirkon, Titan und Nioboxid aktiviert werden,
- Ausführen der Umwandlung des Kohlendioxids durch Wasser bei einer konstanten Temperatur von 280°-295°C und einem konstanten Druck von 27MPa, so dass sich als Zielprodukt der Flüssigphase in einer Trennvorrichtung Ameisensäure mit einer Selektivität von mehr als 95 % ergibt.

2. Verfahren nach Anspruch 1,
**dadurch gekennzeichnet,**
**dass** dem Empfang des Flüssigprodukts die Erzeugung von Wasserstoff und Methan in einem Betrag von 0,63-0,68 sowie 0,07-0,123 mol per mol per mol des umgewandelten Kohlenstoffs folgt.

3. Verfahren nach Anspruch 1,
**dadurch gekennzeichnet,**
**dass** der heterogene Katalysator auf der Basis von Kupfer, Zink und Aluminiumoxiden in einem Massenverhältnis von (50-63)/(35-20)/(2-30), vorzugsweise (50-56)/(28-24)/(16-26), steht, wobei diese Stoffe von einem festen oder flexiblen Träger in der Form von Membranen getragen werden und durch Blei, Zirkon, Titan und Nioboxiden in folgendem Verhältnis aktiviert werden: (50-65)/(20-30)/(20-30)/(0,5-4,5)), vorzugsweise (58-60)/(20-22)/(15,5-20)/(2-2,5).

4. Verfahren nach Anspruch 1,
**dadurch gekennzeichnet,**
**dass** die wässrige Lösung des Kohlendioxids mit einer Massenkonzentration von 3-12 % Kohlendioxid der Reaktionsvorrichtung im Massenverhältnis von Wasser/Kohlenstoff/Katalysator zu (20-50)/(0,5-4)/1 zugeführt wird.

## Revendications

1. Procédé d'utilisation de gaz carbonique par le biais de carbonisation catalytique de solutions aqueuses contenant ce qui suit:
- la pression du dioxyde de carbone initiale est supérieure à la valeur critique,
- le mélangeage du dioxyde de carbone sous pression avec de l'eau supercritique,
- le chauffage du mélange à la température supercritique de 280° vers 295°C,
- l'alimentation du mélange en façon continue en un dispositif de réaction porté par un catalyseur hétérogène sur la base de cuivre, de zinc et d' oxyde d'aluminium sur un support fixe ou flexible sous forme de membranes et activé par le plombe, le zirconium, le titan et l'oxyde de niobium,
- l'exportation de la conversion du dioxyde de carbone par l'eau en une température constante de 280°-295°C et une pression constante de 27MPa de sorte que le produit-cible de la phase liquide en un dispositif de séparation est l'acide formique présentant une sélectivité de plus de 95 %.

2. Procédé selon la revendication 1
Est caractérisé de sorte que
La production d'hydrogène et de méthane suit la réception du produit liquide en une somme de 0,63-0,68 ainsi qu'en une somme de 0,07-0,123 mol per mol per mol du carbone converti.

3. Procédé selon la revendication 1
Est caractérisé de sorte que
Le catalyseur hétérogène est placé sur la base de cuivre, de zinc et d'oxyde d'aluminium dans un rapport massique de (50-63)/(35-20)/(2-30), de préférence (50-56)/(28-24)/(16-26), où ces matières sont portées par un support fixe ou flexible sous forme de membranes et sont activées par le plomb, le zirconium, le titan et l'oxyde de niobium dans le rapport suivant : (50-65)/(20-30)/(20-30)/(0,5-4,5)), de préférence (58-60)/(20-22)/(15,5-20)/(2-2,5).

4. Procédé selon la revendication 1
Est caractérisé de sorte que
La solution aqueuse du dioxyde de carbone est acheminée avec une concentration massique de 3-12 % dioxyde de carbone du dispositif de réaction dans le rapport massique eau/carbone/catalyseur à (20-50)/(0,5-4)/1.
